# EUROPÄISCHE PATENTSCHRIFT

(11) **EP 1 235 912 B1**
(45) Veröffentlichungstag und Bekanntmachung des Hinweises auf die Patenterteilung: **11.04.2012**
(21) Anmeldenummer: 00960654.2
(22) Anmeldetag: 13.09.2000
(51) Int. Cl.: A61P 35/00

(54) **TUMORSPEZIFISCHER VEKTOR FÜR DIE GENTHERAPIE**
TUMOUR-SPECIFIC VECTOR FOR GENE THERAPY
VECTEUR SPECIFIQUE DE TUMEURS DESTINE A LA THERAPIE GENIQUE

(30) Priorität: 04.10.1999 DE 19947668
(43) Veröffentlichungstag der Anmeldung: 04.09.2002
(73) Patentinhaber: Heart BioSystems GmbH, 69120 Heidelberg (DE)
(72) Erfinder: KÜPPER, Jan-Heiner, 47574 Goch (DE); MEYER, Ralph, 55596 Waldböckelheim (DE); MEYER-FICCA, Mirella, 72810 Gomaringen (DE); KANDOLF, Reinhard, 72379 Hechingen (DE)
(74) Vertreter: Simandi, Claus
(86) Internationale Anmeldenummer: PCT/EP2000/008921
(87) Internationale Veröffentlichungsnummer: WO 2001/025441

(56) Entgegenhaltungen:
- WO-A-00/56909
- WO-A-96/18737
- WO-A-98/11207
- WO-A-98/35028
- WO-A-99/60142
- DE-A- 19 757 984
- US-A- 5 457 035
- US-A- 5 856 153
- TOMANIN R. ET AL.: "Development and characterization of a binary gene expression system based on bacteriophage T7 components in adenovirus vectors" GENE, Bd. 193, Nr. 2, 9. Juli 1997 (1997-07-09), Seiten 129-140, XP004093200 ISSN: 0378-1119
- NETTELBECK D. M. ET AL.: "Cell cycle regulated promoters for the targeting of tumor endothelium." ADV. EXP. MED. BIOL., Bd. 451, 1998, Seiten 437-440, XP000979223
- PAN C.-X. ET AL.: "A novel tumor-specific gene therapy for bladder cancer." MEDICAL HYPOTHESES, Bd. 53, Nr. 2, August 1999 (1999-08), Seiten 130-135, XP000978673 ISSN: 0306-9877
- JOKI T. ET AL.: "ACTIVATION OF THE RADIOSENSITIVE EGR-1 PROMOTER INDUCES EXPRESSION OF THE HERPES SIMPLEX VIRUS THYMIDINE KINASE GENE AND SENSITIVITY OF HUMAN GLIOMA CELLS TO GANCICLOVIR" HUMAN GENE THERAPY, Bd. 6, Dezember 1995 (1995-12), Seiten 1507-1513, XP002922862 ISSN: 1043-0342 in der Anmeldung erwähnt
- KOGA S. ET AL.: "A novel telomerase-specific gene therapy: Gene transfer of caspase-8 utilizing the human telomerase catalytic subunit gene promoter." HUMAN GENE THERAPY, Bd. 11, Nr. 10, 1. Juli 2000 (2000-07-01), Seiten 1397-1406, XP000979216 ISSN: 1043-0342
- NETTELBECK D. M. ET AL.: "Gene therapy: Designer promoters for tumour targeting." TRENDS IN GENETICS, Bd. 16, Nr. 4, April 2000 (2000-04), Seiten 174-181, XP002158632 ISSN: 0168-9525
- TAKAKURA: "Cloning of human telomerase catalytic subunit (hTERT) gene promoter and identification of proximal core promoter sequences essential for transcriptional activation in immortalized and cancer cells", CANCER RES, vol. 59, 1 February 1999 (1999-02-01), pages 551-557,

## Beschreibung

Die vorliegende Erfindung betrifft einen Vektor zur gentherapeutischen Behandlung von Tumoren, insbesondere im Zusammenhang mit einer strahlentherapeutischen Behandlung, dessen DNA-Sequenz zumindest einen gewebespezifischen Promotor und zumindest ein therapeutisches Gen aufweist, dessen Expression durch den Promotor kontrolliert wird.

Ein derartiger Vektor ist aus der DE 44 44 949 C1 bekannt.

Unter "Tumoren" werden im Rahmen der vorliegenden Erfindung sowohl maligne als auch benigne Tumore verstanden.

Ca. 30 % der Todesfälle in den zivilisierten Ländern gehen auf maligne Tumorerkrankungen zurück, wobei es trotz weltweiter jahrzehntelanger Anstrengungen derzeit noch für keinen Tumor eine sichere Therapie gibt. Viele Tumoren lassen sich überhaupt nicht oder nur sehr schlecht therapieren.

Zu den benignen Tumoren zählen z.B. Gefäßwandtumore, für die z.T. dieselben Therapien eingesetzt werden wie zur Behandlung maligner Tumoren, also bei Krebs. Derartige Gefäßwandtumore entstehen als Rezidivstenose im wesentlichen durch Proliferationsinduktion von glatten Muskelzellen in der Gefäßwand als Folge von sogenannten Ballondilatationen (PTCA) zur Therapie örtlicher Gefäßwandstenosen, die für die Blutversorgung eines Organs limitierend sein können. Neben der Ballondilatation werden derartige arteriosklerotische Erkrankungen auch noch durch Bypass-Operationen, Stents und weitere alternative Therapieverfahren behandelt, bei denen es jedoch ebenfalls das Problem der sogenannten Rezidivstenose gibt, also die Verengung des Lumens des behandelten Gefäßes durch einen gutartigen Tumor.

Neben der chirurgischen Entfernung von sowohl malignen als auch benignen Tumoren und deren Behandlung mit Zytostatika stellt die Strahlentherapie aus heutiger Sicht eine der wichtigsten Säulen der Tumortherapie dar. Die Tumorvernichtungswahrscheinlichkeit hängt dabei von der applizierten Dosis ab, wobei die zu applizierende Dosis durch die Strahlenempfindlichkeit des unweigerlich mit bestrahlten Normalgewebes limitiert ist. Therapeutische Erfolge hängen somit von der relativen Strahlenempfindlichkeit der Tumorzellen im Vergleich zu den Zellen des Nachbargewebes ab.

Eine Erhöhung der therapeutischen Breite durch selektive Strahlensensibilisierung von Tumorzellen würde folglich einen signifikanten Fortschritt bei der Behandlung von Tumoren bedeuten und zu einer Verbesserung der Heilungsraten führen. Aus diesem Grund werden in der Klinik bereits pharmakologische Radiosensitizer eingesetzt, mit denen die Tumorzellen empfindlicher gegen Bestrahlung gemacht werden sollen.

Die Gentherapie bietet erstmals die Möglichkeit, signifikante Fortschritte bei der Krebsbekämpfung dadurch zu erzielen, daß therapeutische Gene zur Verstärkung der Strahlen- oder Zytostatika-Therapie eingesetzt werden können.

Dabei spielen virale Vektorsysteme zur Transduktion therapeutischer Gene eine große Rolle. Neben retroviralen Vektorsystemen, die eine gewisse Präferenz für proliferierende Zellen haben und sehr häufig in das zelluläre Genom integrieren, werden vor allem adenovirale Vektorsysteme diskutiert, mit denen ein hoher Viruspartikeltiter erreichbar ist, die eine gute Transduktionseffizienz und sehr geringe Integrationsrate aufweisen; siehe Ali et al., Gene Ther. (1991), Band 1, 367-384.

Entscheidend für eine sichere Gentherapie ist dabei, daß das therapeutische Gen nur in den gewünschten Zielzellen zum Einsatz kommt. Wünschenswert bei der Tumorgentherapie wäre daher der Einsatz eines Tumorzell-spezifischen Promotors, der Aktivität in verschiedenen Tumorspezies und Nichtaktivität in allen Arten von Normalgewebe zeigt. Ein derartiger Promotor wurde bisher jedoch noch nicht gefunden.

Die eingangs erwähnte DE 44 44 949 C1 beschreibt in diesem Zusammenhang einen Vektor für die gentherapeutische Behandlung von Patienten, bei denen nach operativer Entfernung eines Tumors eine Nachbehandlung mit konventionellen Bestrahlungs- und/oder Zytostatikaverfahren erfolgt. Der Vektor enthält hinter einem in Tumorzellen aktiven Promotor eine exprimierbare Insert-DNA, die für die DNA-Bindungsdomäne (DBD) einer Poly (ADP-Ribose)-Polymerase (PARP) kodiert. Die zugrunde liegende Idee bei dieser Veröffentlichung besteht darin, das zur Reparatur von DNA-Schädigungen benötigte Enzym PARP durch Zugabe von DBD-Molekülen in seiner Aktivität zu hemmen, so daß die Reparatur von DNA-Schäden verhindert wird.

Als Beispiel für einen gewebespezifischen Promotor ist der P4-Promotor von MVM genannt. Der Vektor kann ein viraler Vektor sein, wobei die Viren replikationsinkompetent sind und *in trans* komplementiert werden können, um Viren zu erhalten, die für DBD kodieren, sich im Patienten jedoch nicht vermehren können.

Bei dem bekannten Vektor hat sich als Nachteil herausgestellt, daß der P4-Promotor noch nicht die für eine sichere Gentherapie erforderliche Gewebespezifität aufweist, so daß auch bei Normalgewebe eine Hemmung der DNA-Reparatur auftritt, was aus nicht weiter zu erläuternden Gründen unerwünscht ist.

Die meisten gentherapeutischen Ansätze beinhalten deshalb eine Verstärkung der Tumor-spezifischen Immunantwort, sind also a priori systemisch, da die lokale Verstärkung der Immunantwort nicht auf den Tumor begrenzt bleibt. Dies ist zugleich auch ein gewünschter Vorteil, denn mit dieser Therapie lassen sich prinzipiell auch Metastasen abtöten.

Um eine mehr oder weniger lokale Gentherapie zu ermöglichen, wurde bereits der Einsatz von Retroviren diskutiert, die eine Gentransduktion nur in teilende Zellen vornehmen. Ferner wurden Adenovirusmutanten vorgeschlagen, die nur in p53-negativen Tumorzellen replizieren sollen; Heise et al., Nat. Med. (1997), Band 3, 639-645. Hierbei wird davon ausgegangen, daß alle p53-positiven Zellen die Proliferation des Adenovirus verhindern können.

Darüber hinaus wurde bereits die Verwendung Strahlunginduzierbarer Promotoren durch Joki et al., Hum. Gene Ther. (1995), Band 6, 1507-1513, sowie Zellzyklus-spezifischer Promotoren durch Nettelbeck et at., Adv. Exp. Med. Biol. (1998), Band 451, 437-440 vorgeschlagen.

All diese bekannten Lösungen sind jedoch mit spezifischen Nachteilen verbunden. Das Konzept der Immuntherapie hat den prinzipiellen Nachteil, daß immunsupprimierte Patienten darauf nicht ansprechen können, wobei das Immunsystem dieser Patienten oft durch die Tumorerkrankung selbst zusätzlich angeschlagen ist. Weiter hat sich gezeigt, daß ein immuntherapeutisches Gen oder ein sogenanntes Suizidgen, das zur Abtötung der Zielzelle führt, allein für *in vivo*-Applikationen nicht ausreicht; Uckert et al., HUM. GENE THER. (1998), Band 9, 855-865. Auch die Kombination aus mehreren immunstimulierenden Genen gegebenenfalls im Zusammenhang mit Suizidgenen hat bisher nicht zu einem Durchbruch geführt, der eine Standardanwendung bei einer gegebenen Tumorerkrankung erlauben würde.

Die oben erwähnten Retroviren haben ferner keine ausschließliche Selektivität für Tumoren, sondern infizieren alle teilenden Zelltypen, sofern ein entsprechender Rezeptor vorhanden ist. Die Infektion einer gesunden Zelle birgt zudem das Risiko der Insertionsmutagenese, da Retroviren in das zelluläre Genom integrieren. Auch replikationskompetente Adenoviren, die für eine gezielten Tumortherapie vorgeschlagen wurden, sind nicht spezifisch für Tumorzellen, wie sich inzwischen herausgestellt hat, wobei der Effekt auch unabhängig vom p53-Status ist; Rothmann et al., J. Virol. (1998), Band 72, 9470-9478.

Auch die bisher beschriebene Verwendung von Suizidgenen weist Nachteile auf, da diese die Nebenwirkungen in gesunden proliferativen Geweben erhöhen. Dieses Problem könnte nur dann vermieden werden, wenn die Suizidgene Tumor-spezifisch exprimiert würden.

Pan (1999); Med. Hypoth. 53(2): 130-135 beschreibt die Eignung eines hTERT-Promotors in einem therapeutischen Retrovirus für ein gentherapeutisches Verfahren, jedoch ohne ein erforderliches geeignetes Steuerungssystem zu offenbaren.

Vor diesem Hintergrund liegt der vorliegende Erfindung die Aufgabe zugrunde, einen Vektor der eingangs genannten Art zu schaffen, der Tumor-spezifisch wirkt und nur geringe Nebenwirkungen auf Normalgewebe aufweist.

Bei dem eingangs erwähnten Vektor wird diese Aufgabe erfindungsgemäß nach Anspruch 1 gelost.

Die der Erfindung zugrunde liegende Aufgabe wird auf diese Weise vollkommen gelöst.

Die Erfinder der vorliegenden Anmeldung haben nämlich erkannt, daß der Promotor für die katalytische Untereinheit der Telomerase besonders gut für eine Tumor-spezifische Gentherapie einsetzbar ist. Dieser Promotor, der z.B. beschrieben wurde von Horikawa et al., Cancer Res. (1999), Band 59, 826-830, sowie Takakura et al., Cancer Res. (1999), Band 59, 551-557, hat nämlich eine hohe Aktivität in Tumorzellen, mehr als 90 % aller Tumoren sind positiv für Telomerase. Ferner weist er eine schwächere Aktivität in einigen wenigen proliferativen Stammzellen, z.B. Keimbahnzellen und Blutstammzellen, sowie keine Aktivität in den weitaus meisten proliferativen Zelltypen auf, wie z.B. Endothelzellen, Fibroblasten, Hepatozyten etc.

Aus der WO 98/11207 ist es in diesem Zusammenhang bekannt, einen Telomerase-Promotor im Zusammenhang mit der Transfektion von Zellen zu verwenden, um Produkte zu exprimieren, die im Hinblick auf eine Krebsbehandlung das Zellwachstum inhibieren. Die Anwendung eines Telomerase-Promotors im Zusammenhang mit einer strahlentherapeutischen Behandlung wird in dieser Druckschrift jedoch nicht erwähnt.

Neben der Sicherheit, die durch die Tumor-spezifischen Promotoren für die katalytische Untereinheit der Telomerase gegeben ist, ergibt sich eine zweite Sicherheit durch die Anwendung dieser Promotoren bzw. der durch sie kontrollierten therapeutischen Gene im Zusammenhang mit einer Strahlentherapie, die nach dem heutigen Stand der Technik sehr lokal nur auf das Tumorgewebe ausgerichtet sein kann.

Durch die Kombination verschiedener therapeutischer Gene, die gewebespezifisch exprimiert werden, und der lokal gerichteten Strahlentherapie kann dafür gesorgt werden, daß Tumorzellen,wenn auch vielleicht nur um einen geringen Faktor, empfindlicher reagieren als das Nachbargewebe, wodurch der Tumor dann jedoch effizient abgetötet werden kann.

Neben der Verwendung des neuen Vektors bei der Strahlentherapie ist auch die Verwendung im Zusammenhang mit der Chemotherapie von Vorteil, denn auf diese Weise können nichtlokalisierte Metastasen abgetötet werden.

Dabei ist es bevorzugt, wenn das therapeutische Gen für ein Protein kodiert, das ausgewählt ist aus der Protein-Gruppe: Cytosindeaminase (CD), Herpes-Simplex-Virus-Thymidinkinase (HSV-TK), DNA-Bindungsdomäne (DBD) der Poly(ADP-Ribose)-Polymerase (PARP), zytotoxische Protease 2A bzw. 3C von Picornaviren, vorzugsweise von Enteroviren, weiter vorzugsweise von Coxsackieviren der Gruppe B (CVB), insbesondere des Serotyps B3.

Cytosindeaminase wandelt 5-Fluorcytosin in 5-Fluoruracil um, das in die DNA replizierender Zellen eingebaut wird und diese dann abtötet. Eine sytemische 5-Florcytosin-Behandlung im Zusammenhang mit lokaler Strahlentherapie führt zu einer gezielten Verstärkung der Tumorabtötung, da nur in den Tumorzellen die Cytosindeaminase gebildet wird, so daß die gefürchteten Nebenwirkungen wie Nekrosen/Fibrosen im Nachbargewebe, Schädigung des Knochenmarks und der Darmschleimhaut etc. vermieden werden.

Auf ähnliche Weise wirkt auch die HSV-TK, dieses Enzym aktiviert Ganzyklovir, das sich ebenfalls in die DNA replizierender Zellen einbaut und die DNA zerstört, so daß im Zusammenhang mit lokaler Strahlentherapie die gleichen Vorteile wie mit CD erreicht werden.

Im Gegensatz zu CD und HSV-TK führt die Expression von DBD-Molekülen dazu, daß die Aktivität von PARP gehemmt wird, das zur Reparatur von DNA-Schädigungen benötigt wird. Auf diese Weise können im Zusammenhang mit der lokalen Strahlentherapie "vorgeschädigte" Tumorzellen nicht wieder "repariert" werden, so daß sie absterben.

Die Proteasen 2A und 3C induzieren dagegen die Apoptose in Zellen und sind daher zytotoxisch.

Die Erfinder der vorliegenden Anmeldung haben nun erkannt, daß sich durch die Kombination von Strahlentherapie einerseits sowie von durch die obgengenannten Promotoren gewebespezifisch exprimierten Proteinen der vorstehend genannten Art eine selektive Tumorabtötung erreichen läßt, die deutlich über den Wirkungen der bisher eingesetzten Einzelmaßnahmen liegt.

Dabei ist es bevorzugt, wenn das therapeutische Gen ein Fusionsgen ist, das für ein Fusionsprotein aus zumindest zwei Proteinen kodiert, die aus der oben erwähnten Protein-Gruppe ausgewählt sind, wobei das therapeutische Gen vorzugsweise zwischen den Sequenzbereichen für die beiden Proteine für einen Peptidlinker kodiert, der vorzugsweise Glyzin, insbesondere 8-10 Glyzine umfaßt.

Hier ist von Vorteil, daß sich ein synergistischer Effekt erreichen läßt, wenn zwei Suizidgene in dem therapeutischen Gen enthalten sind, wobei insbesondere bei unterschiedlichen Wirkmechanismen der Partner innerhalb der Fusionsproteine eine synergistische Wirkung entsteht. Der Vorteil der Expression von Fusionsgenen liegt in der Möglichkeit, zwei unterschiedliche therapeutische Prinzipien simultan zu übertragen und damit additive oder häufig sogar synergistische Effekte in der Therapie zu erzielen. Damit die Proteindomänen der Fusionspartner sich für die Anwendung optimal falten können, kann es sinnvoll sein, die Information für einen kurzen Peptidlinker, vorzugsweise 8-10 Glyzine, zwischen die cDNAs zu klonieren.

Besonders bevorzugt sind die folgenden Fusionsproteine:
CD-Linker-HSV-TK, CD-Linker-DBD, CD-Linker-2A, CD-Linker-3C, HSV-TK-Linker-DBD, HSV-TK-Linker-2A, HSV-TK-Linker-3C, DBD-Linker-2A und DBD-Linker-3C. Die Reihenfolge der Fusionspartner innerhalb eines Fusionsproteins kann auch entgegengesetzt sein.
Insgesamt ist es bevorzugt, wenn der Vektor auf einem Virus-Vektor beruht, insbesondere auf einem Adenovirus-Vektor oder einem Adeno-assoziierten Virus-Vektor (AAV).

Ferner betrifft die Erfindung einen Retrovirus-Vektor, der für den neuen Vektor kodiert.

Das neue Gentherapiesystem kann also sowohl mit konventionellen viralen oder nicht-viralen Vektoren als auch über einen Retrovirus-Vektor in den Körper eingebracht werden, wo er sich wesentlich selektiver auf den Tumor auswirkt, als dies bisher möglich war.

Wenn zwischen dem Promotor und dem therapeutischen Gen ein positiv rückgekoppeltes System vorgesehen ist, das von dem Promotor angetrieben wird und selbst die Expression des therapeutischen Gens kontrolliert, wobei das positiv rückgekoppelte System vorzugsweise den T7-Promotor sowie das Gen für die T7-RNA-Polymerase umfaßt, und weiter vorzugsweise der Promotor das Gen für die T7-RNA-Polymerase und der T7-Promotor das therapeutische Gen kontrolliert, wobei ferner eine zweite Expressionseinheit vorgesehen ist, die die T7-RNA-Polymerase unter der Kontrolle des T7-Promotors enthält.

Durch dieses System positiver Rückkopplung wird für eine verstärkte Expression des therapeutischen Gens in den Zielzellen gesorgt, wobei der Promotor, also der Telomerase- Promotor dafür sorgt, daß das positiv rückgekoppelte System nur in den Zielzellen "angestoßen" wird. Bei dieser positiven Rückkopplung wird von der Erkenntnis ausgegangen, daß der T7-Promotor in eukariontischen Zellen ohne die T7-RNA-Polymerase stumm ist, so daß ein sehr sicheres System vorliegt, das den therapeutischen Effekt potenziert, ohne Nebenwirkungen zu zeigen.

In einer ersten Expressionseinheit kontrolliert der Telomerase-Promotor die Expression der T7-RNA-Polymerase. Die zweite Expressionseinheit enthält dann den T7-Promotor, der wiederum die Expression der T7-RNA-Polymerase kontrolliert. Auf diese Weise wird die Produktion der T7-RNA-Polymerase mit positiver Rückkopplung verstärkt. Weil nun in der dritten Expressionseinheit das therapeutische Gen unter Kontrolle des T7-Promotors ist, wird durch die positiv rückgekoppelte Expression der T7-RNA-Polymerase auch die Expression des therapeutischen Genes verstärkt.

Weitere Merkmale und Vorteile der Erfindung ergeben sich aus der nachfolgenden Beschreibung bevorzugter Ausführungsbeispiele.

Die nachstehenden Beispiele werden anhand der beigefügten Zeichnung näher erläutert, in der:
- Fig. 1: virale Vektoren zur Expression therapeutischer Gene in Tumorzellen zeigt; und
- Fig. 2: virale Vektoren mit positiv rückgekoppelter Expression therapeutischer Gene in Tumorzellen zeigt.

### Beispiel 1: Generierung der Bausteine für die viralen Vektoren

1.1 Telomerase-Promotor: Die Sequenz für den Telomerase-Promotor ist bekannt; Horikawa et al., a.a.O. und Takakura et al., a.a.O. Der Promotor kann z.B. mit folgenden Primern über PCR aus Zellen (z.B. HeLa-Tumorzellen) amplifiziert werden:

| | |
|---|---|
| Vorwärtsprimer: | ATC AGC TTT TCA AAG ACA CAC |
| Rückwärtsprimer: | CGC GGG GGT GGC CGG GGC CAG |

1.2 T7-Promotor: Der Promotor für die T7-RNA-Polymerase (ca. 30 bp) kann durch BssHI/Kpnl-Verdau aus dem Plasmid pCR-Script (Stratagene) erhalten werden. Die Sequenzen von T7-Promotor und -RNA-Polymerase sind z.B. beschrieben von Dunn und Studier, J Mol Biol (1983), Band 166, 477-535; GenBank Accession No. V01146, J02518, X00411.
1.3 Cytosindeaminase (CD): Das Gen für die CD (1,3 kb) kann durch BamHI/Notl-Verdau aus dem Plasmid pcDNA3-CD der Anmelderin gewonnen werden. Die CD-Sequenz wurde z.B. beschrieben von Austin und Huber, Mol. Pharmacol. (1993), Band 43, 380-387; GenBank Accession No. S56903.
1.4 Herpes-Simplex-Virus-Thymidinkinase (HSV-TK): Das Gen für die HSV-TK (1,1 kb) kann durch BamHI/BgII-Verdau aus dem Plasmid pCDTK der Anmelderin gewonnen werden. Die HSV-TK-Sequenz beschreiben Suzutani et al. in Microbiol. Immunol. (1995), Band 39, 787-794; GenBank Accession No. AB009255.
1.5 DNA-Bindungsdomäne (DBD) der Poly(ADP-Ribose)-Polymerase (PARP): Die DBD (1,1 kb) kann durch Xbal/Sall-Verdau aus dem Plasmid pPARP6 gewonnen werden; Küpper et al., J. Biol. Chem. (1990), Band 265, 18721-18724.
1.6 CVB3-Protease 2A: Die kodierenden Sequenzen für die zytotoxische Protease 2A wird durch Pme-Verdau aus dem Plasmid pIND-2A der Anmelderin gewonnen.
1.7 CVB3-Protease 3C: Die kodierende Sequenz für die zytotoxische Protease 3C wird durch Pme-Verdau aus dem Plasmid pIND-3C der Anmelderin gewonnen.
Klump et al. beschreiben in J. Virol. (1990), Band 64, 1573-1583 die komplette Sequenz für eine CVB3 cDNA; GenBank Accession No. M33854; Protein 2A: Nukleotid 3304-3744; Protein 3C: Nukleotid 5362-5910.
1.8 Fusionsgene: Unter einem Fusionsgen wird die durchgehende Sequenz eines therapeutischen Genes aus mehreren, vorzugsweise zwei cDNAs verstanden, die von einem einzigen Promotor zu einem zusammenhängenden Fusionsprotein exprimiert werden. Auf diese Weise können zwei therapeutische Prinzipien simultan übertragen werden, so daß sich additive oder synergistische Effekte in der Therapie ergeben.
Damit die Proteindomänen der Fusionspartner sich für die Anwendung optimal falten können, wird die Information für einen kurzen Peptidlinker, vorzugsweise für Glyzin, insbesondere 8-10 Glyzine, zwischen die cDNAs kloniert.
Auf diese Weise werden die folgenden Fusionsgene erzeugt:
CD-Linker-HSV-TK, CD-Linker-DBD, CD-Linker-2A, CD-Linker-3C, HSV-TK-Linker-DBD, HSV-TK-Linker-2A, HS-TK-Linker-3C,
DBD-Linker-2A und DBD-Linker-3C. Die Reihenfolge der Fusionspartner innerhalb eines Fusionsgenes kann auch entgegengesetzt sein.

1.9 T7-RNA-Polymerase (T7 Pol): Die cDNA für die T7 Pol (2,6 kb) stammt aus dem Plasmid pAR3132 der Anmelderin und enthält die Codons 11-883 des T7-RNA-Polymerase-Gens.

Die Sequenzen der Bausteine sind in der Genbank PubMed der National Library of Medicine (http://ww4.ncbi.nlm.nih.gov/PubMed/) abgelegt.

### Beispiel 2: Herstellung von rekombinaten Adenoviren

Zur Herstellung rekombinanter Adenoviren wird beispielsweise das E1/E3-deletierte Adenovirus-5-System von Vogelstein verwendet; He et al., PNAS (1998), Band 95, 2509-2514.

Die zu exprimierende cDNA, also das therapeutische Gen, wird in den Vektor pShuttle (6,7 kb) kloniert. Vor die cDNA wird der für die Anwendung vorgesehene Promotor (Telomerase) kloniert, hinter die cDNA ein Poly-Adenylierungssignal. Das neu entstandene Plasmid wird zusammen mit dem Helferplasmid pAdEasyl in den Rekombinations-kompetenten Bakterienstamm BJ5183 transformiert.

Nach homologer Rekombination der überlappenden Sequenzen von Shuttle- und Helfervektor entsteht ein rekombinantes Adenovirusgenom, das aus den Bakterien isoliert und zur präparativen Aufbereitung in den RecA⁻-Stamm HB101 transformiert wird. Plasmid-Material wird danach aus diesen Baktieren im präparativen Maßstab isoliert und über Cäsiumchlorid-Zentrifugation gereinigt.

Das dann erhaltene Plasmid-Material wird in die E1-exprimierende Helferzellinie 911 transfiziert; Fallaux et al., Hum. Gene Ther. (1996), Band 7, 215-222. Nach Transfektion des rekombinanten Adenovirusgenoms werden die Zellen mit Soft-Agar überschichtet. In transfizierten Zellen kommt es dann zur Virusvermehrung, und es entsteht ein Plaque, aus dem die rekombinanten Viren durch Gefrier-Tau-Lyse isoliert werden können. Nach zwei Tagen kann Expression nachgewiesen werden, es tritt aber noch kein zytopathischer Effekt (CPE) auf. Zur Gewinnung von Virus-Stocks werden neue 911-Zellen mit den entsprechenden rekombinanten Adenoviren infiziert. Nach ca. vier Tagen ist der CPE voll ausgebildet. Die Zellen werden in einem Dounce-Homogenisator aufgeschlossen, Zell-Trümmer werden durch kurze Zentrifugation pelletiert, und die im Überstand befindlichen Viren werden über Cäsium-Chlorid-Dichtezentrifugation gereinigt.

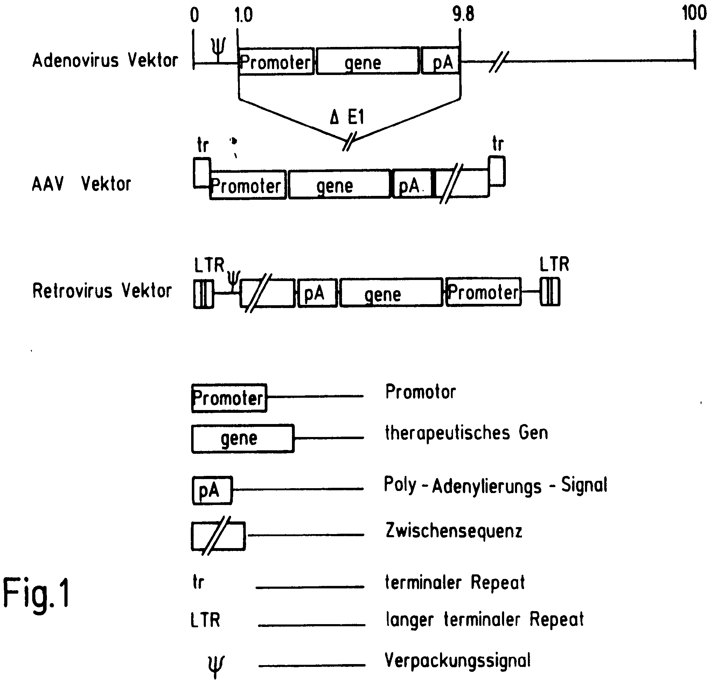

Der so gewonnene Adenovirus-Vektor ist in Fig. 1 oben dargestellt, die verwendeten Abkürzungen sind in der Figurenlegende erklärt.

### Beispiel 3: Herstellung von rekombinanten Adeno-assoziierten Viren (AAV)

Hier wird beispielsweise das aus zwei Plasmiden plus Adenovirus bestehende System von Samulski verwendet; Snyder et al.: "Production of recombinant adeno-associated viral vectors". Current Protocols in Human Genetics. New York: John Wiley and Sons (1996), 12.1.1-12.1.24.

Die zu exprimierende cDNA, also das therapeutische Gen, wird zusammen mit regulatorischen Sequenzen (Promotor und Poly-A-Signal) in einen Vektor kloniert, der nur die terminalen Repeats von AAV-2 enthält. Diese Repeats sind die minimalen Cisregulatorischen Sequenzen, die für Replikation und Verpackung benötigt werden; Xiao et al., J. Virol. (1997), Band 71, 941-948.

Der Vektor entsteht, indem aus dem Plasmid pSub201 (Human Gene Therapy Center, University of North Carolina, Chapel Hill, NC, USA) über Xbal-Verdau die Rep/Cap-Sequenz excisiert wird. Die terminalen Repeats von jeweils 0,18 kb verbleiben im Vektor.

In diesen Vektor werden anschließend die für das jeweilige Gentransfersystem vorgesehenen Bausteine, wie Promotor, cDNA für das therapeutische Gen und Poly-Adenylierungssignal kloniert. Die dabei entstehenden Vektorplasmide werden jeweils zusammen mit dem Helferplasmit pAAV/Ad (Human Gene Therapy Center), das die Struktur- und Nichtstrukturproteine (cap und rep) von AAV *in trans* zur Verfügung stellt, in 293-Zellen transfiziert. Am nächsten Tag wird Wildtyp-Adenovirus als Helfer für die AAV-Replikation mit einer MOI von 3 auf die Zellen gegeben. Zwei bis drei Tage nach Transfektion/Infektion ist der zytopathische Effekt (CPE) gut sichtbar, und rekombinantes AAV kann aus den Zellen gewonnen werden.

Die Methode der Cäsium-Chlorid-Reinigung erfolgt wie von Snyder et al. a.a.O. beschrieben. Wesentliche Elemente dieser AAV-Reinigung sind dreimalige Gefrier-Tau-Lyse der infizierten Zellen plus Ultrabeschallung zur Freisetzung der Viren, AmmoniumSulfat-Präzipitation zur Entfernung zellulärer Proteine, Reinigung der AAV-Partikel auf einem CsC1-Gradienten durch Ultrazentrifugation, Dialyse der gereinigten AAV-Fraktionen durch PBS und Hitze-Inaktivierung von kontaminierenden Adenoviren durch Inkubation bei 56 °C für 15 Minuten (AAV wird durch diese Behandlung nicht inaktiviert).

Der so gewonnene AAV-Vektor ist in Fig. 1 in der Mitte dargestellt.

### Beispiel 4: Herstellung rekombinanter Retroviren

Hier kann beispielsweise das System der Firma Clonetech (Heidelberg) verwendet werden. Dieses besteht aus Shuttle-Vektoren, beispielsweise pLNCX (6,2 kb), die man über Transformation in Bakterien vermehren kann, sowie einer Helferzellinie, die RetroPack PT67 Linie, die eine Transkomplementation der Vektoren zu Virionen ermöglicht.

Vor der Verwendung des Shuttle-Vektors pLNCX wird der CMV-Promotor aus diesem Vektor entfernt, um dann durch den Promotor der Wahl, also dem Telomerase-Promotor , ersetzt zu werden. In die multiple Klonierungssequenz werden dann die oben erwähnten therapeutischen Gene bzw. Fusionsgene mit Poly-Adenylierungssignalen kloniert.

Der rekombinante Vektor wird in die Verpackungszellinie PT67 transfiziert. Mit dem Antibiotikum G418 kann auf transfizierte Zellen selektioniert werden. Es kommt zur Produktion rekombinanter Retroviren, die über analoge Verfahren, wie oben in Beispiel 2 und 3 beschrieben, aus Zellen und Zellkulturüberstand gewonnen werden und mit Cäsium-Chlorid-Dichtezentrifugation gereinigt werden können.

Der so gewonnene Retrovirus-Vektor ist prinzipiell in Fig. 1 unten dargestellt.

### Beispiel 5: Adenovirus-Vektor mit positiv rückqekoppeltem System

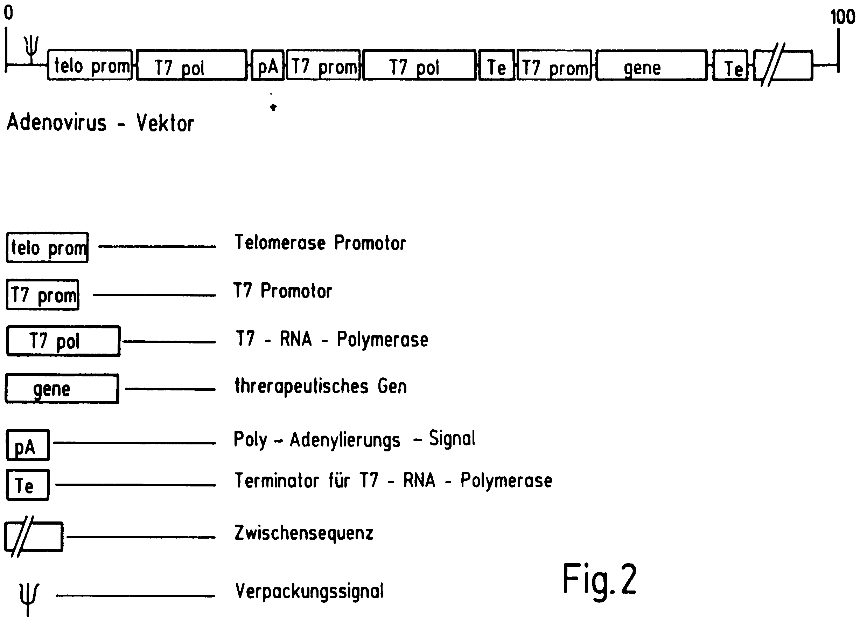

In Fig. 2 ist ein Adenovirus-Vektor dargestellt, der wie in Beispiel 2 beschrieben hergestellt wurde. Statt eines einzelnen Promotors und eines einzelnen therapeutischen Genes enthält dieser Vektor jedoch drei Expressionseinheiten, von denen die erste die T7-RNA-Polymerase unter der Kontrolle des Telomerase-Promotors enthält. Die zweite Expressionseinheit enthält ebenfalls die T7-RNA-Polymerase, jedoch unter der Kontrolle ihres eigenen Promotors, des T7-Promotors. Die dritte Expressionseinheit schließlich enthält das therapeutische Gen unter der Kontrolle des T7-Promotors.

Bei der Infizierung von Zielzellen sorgt zunächst der Telomerase-Promotor für die Expression der T7-RNA-Polymerase (erste Expressionseinheity. Durch die erzeugte T7-RNA-Polymerase wird der T7-Promotor in der zweiten Expressionseinheit "angeschaltet", so daß auch er für die Produktion von T7-RNA-Polymerase sorgt. Hierdurch entsteht ein positiv rückgekoppeltes System, je mehr T7-RNA-Polymerase erzeugt wird, desto mehr T7-Promotor wird angeschaltet.

Auf diese Weise kommt es dann zu einer verstärkten Expression des therapeutischen Genes, das hier unter der Kontrolle eines T7-Promotors steht.

Bei diesem System kontrolliert der Telomerase-Promotor die Expression des therapeutischen Genes also nicht direkt, wie in den Beispielen 2-4, sondern indirekt über das zwischengeschaltete, positiv rückgekoppelte System von T7-Promotor und T7-RNA-Polymerase.

Weil der T7-Promotor in eukariontischen Zellen ohne die T7-RNA-Polymerase, die dort üblicherweise nicht angetroffen wird, stumm ist, kann das therapeutische Gen folglich nur in solchen Zellen exprimiert werden, in denen der Telomerase-Promotor aktiv ist, also vor allem in Tumorzellen.

## Patentansprüche

1. Vektor zur gentherapeutischen Behandlung von Tumoren, **dadurch gekennzeichnet, dass** die DNA-Sequenz umfasst:
a.) einen gewebespezifischen Promotor und zwar einen Promotor für die katalytische Untereinheit der Telomerase,
der ein Gen für die T7-RNA-Polymerase kontrolliert, und
b.) zumindest ein therapeutisches Gen aufweist, wobei ein T7-RNAPromotor das therapeutische Gen kontrolliert, und
c.) ein weitere Expressionseinheit enthaltend ein Gen für die T7-RNA-Polymerase, wobei ein T7-RNAPromotor das Gen für die T7-RNA-Polymerase kontrolliert.

2. Vektor zur gentherapeutischen Behandlung von Tumoren nach Anspruch 1, wobei das therapeutische Gen für ein Protein kodiert, das ausgewählt ist aus der Protein-Gruppe: Cytosindeaminase (CD), Herpes-Simplex-Virus-Thymidinkinase (HSV-TK), DNA-Bindungsdomäne (DBD) der Poly (ADP-Ribose)-Polymerase (PARP), zytotoxische Protease 2A bzw. 3C von Picornaviren, vorzugsweise von Enteroviren, weiter vorzugsweise von Coxsackieviren der Gruppe B, insbesondere des Serotyps B3.

3. Vektor nach Anspruch 2, **dadurch gekennzeichnet, dass** das therapeutische Gen ein Fusionsgen ist, das für ein Fusionsprotein aus zumindest zwei Proteinen kodiert, die aus der Protein-Gruppe ausgewählt sind.

4. Vektor nach Anspruch 3, **dadurch gekennzeichnet, dass** das therapeutische Gen zwischen den Sequenzbereichen für die beiden Proteine für einen Peptidlinker kodiert, der vorzugsweise Glyzin, insbesondere 8-10 Glyzine, umfasst.

5. Vektor nach Anspruch 4, **dadurch gekennzeichnet, dass** das Fusionsgen ausgewählt ist aus der Gruppe: CD-Linker-HSV-TK, CD-Linker-DBD, CD-Linker-2A, CD-Linker-3C, HSV-TK-Linker-DBD, HSV-TK-Linker-2A, HSV-TK-Linker-3C, DBD-Linker-2A und DBD-Linker-3C.

6. Vektor nach einem der Ansprüche 1 bis 5, **dadurch gekennzeichnet, dass** er auf einem Virus-Vektor beruht, insbesondere auf einem Adenovirus-Vektor oder einem Adeno-assozüerten Virus (AAV)-Vektor.

7. Retrovirus, der für einen Vektor nach einem der Ansprüche 1 bis 6 kodiert.

8. Vektor nach einem der Ansprüche 1 bis 6 zur Anwendung in der Behandlung in der Gentherapie.

9. Vektor nach Anspruch 8 zur Anwendung in der Behandlung bei Tumorerkrankungen.

10. Vektor nach Anspruch 9 zur Anwendung in der Behandlung bei Tumorerkrankungen im Zusammenhang mit Strahlentherapie.

11. Vektor nach Anspruch 9 zur Anwendung in der Behandlung bei Tumorerkrankungen im Zusammenhang mit Zytostatikatherapie.

## Claims

1. A vector for treating tumors by gene therapy, **characterized in that** the DNA sequence comprises:
a.) a tissue-specific promoter and, in fact, a promoter for the catalytic subunit of telomerase,
which controls a gene for T7 RNA polymerase, and
b.) at least one therapeutic gene, wherein a T7 RNA promoter controls the therapeutic gene, and
c.) a further expression unit containing a gene for T7 RNA polymerase, wherein a T7 RNA promoter controls the gene for T7 RNA polymerase.

2. The vector for treating tumors by gene therapy according to claim 1, wherein the therapeutic gene codes for a protein that is selected from the group of proteins comprising: cytosine deaminase (CD), herpes simplex-virus thymidine kinase (HSV-TK), DNA-binding domain (DBD) of poly(ADP-ribose) polymerase (PARP), cytotoxic protease 2A and 3C from picornaviruses, preferably of enteroviruses, further preferably from group B Coxsackie viruses, in particular serotype B3.

3. The vector according to claim 2, **characterized in that** the therapeutic gene is a fusion gene coding for a fusion protein comprising at least two proteins selected from the group of proteins.

4. The vector according to claim 3, **characterized in that** the therapeutic gene between the sequence regions for the two proteins codes for a peptide linker which preferably comprises glycine, in particular 8-10 glycines.

5. The vector according to claim 4, **characterized in that** the fusion gene is selected from the group: CD-linker-HSV-TK, CD-linker-DBD, CD-linker-2A, CD-linker-3C, HSV-TK-linker-DBD, HSV-TK-linker-2A, HSV-TK-linker-3C, DBD-linker-2A and DBD-linker-3C.

6. The vector according to one of the claims 1 to 5, **characterized in that** it is based on a virus vector, in particular an adenovirus vector or an adeno-associated virus (AAV) vector.

7. A retrovirus that codes for a vector according to one of the claims 1 to 6.

8. The vector according to one of the claims 1 to 6 for use in treatment in gene therapy.

9. The vector according to claim 8 for use in the treatment of tumors.

10. The vector according to claim 9 for use in the treatment of tumors in association with radiation therapy.

11. The vector according to claim 9 for use in the treatment of tumors in association with cytostatic therapy.

## Revendications

1. - Vecteur pour le traitement de tumeurs par thérapie génique, **caractérisé par le fait que** la séquence d'ADN comprend :
a) un promoteur spécifique de tissu, à savoir un promoteur pour la sous-unité catalytique de la télomérase, lequel contrôle un gène pour l'ARN polymérase T7, et
b) comprend au moins un gène thérapeutique, un promoteur ARN T7 contrôlant le gène thérapeutique, et
c) une autre unité d'expression contenant un gène pour l'ARN polymérase T7, un promoteur ARN T7 contrôlant le gène pour l'ARN polymérase T7.

2. - Vecteur pour le traitement de tumeurs par thérapie génique selon la revendication 1, dans lequel le gène thérapeutique code pour une protéine qui est choisie parmi le groupe de protéines : cytosine désaminase (CD), thymidine kinase du virus de l'herpès simplex (HSV-TK), domaine de liaison à l'ADN (DBD) de la poly(ADP-ribose)-polymérase (PARP), protéase 2A ou 3C cytotoxique de picornavirus, de préférence d'entérovirus, de façon plus préférée de coxsackievirus du groupe B, en particulier du sérotype B3.

3. - Vecteur selon la revendication 2, **caractérisé par le fait que** le gène thérapeutique est un gène de fusion qui code pour une protéine de fusion composée d'au moins deux protéines qui sont choisies parmi le groupe de protéines.

4. - Vecteur selon la revendication 3, **caractérisé par le fait que** le gène thérapeutique code pour un lieur peptidique entre les domaines de séquence des deux protéines, lequel lieur comprend de préférence de la glycine, en particulier 8 à 10 glycines.

5. - Vecteur selon la revendication 4, **caractérisé par le fait que** le gène de fusion est choisi parmi le groupe : CD-lieur-HSV-TK, CD-lieur-DBD, CD-lieur-2A, CD-lieur-3C, HSV-TK-lieur-DBD, HSV-TK-lieur-2A, HSV-TK-lieur-3C, DBD-lieur-2A et DBD-lieur-3C.

6. - Vecteur selon l'une des revendications 1 à 5, **caractérisé par le fait qu'**il consiste en un vecteur viral, en particulier en un vecteur adénoviral ou en un vecteur de virus adéno-associé (AAV).

7. - Rétrovirus qui code pour un vecteur selon l'une des revendications 1 à 6.

8. - Vecteur selon l'une des revendications 1 à 6 pour l'utilisation dans le traitement en thérapie génique.

9. - Vecteur selon la revendication 8 pour l'utilisation dans le traitement de maladies tumorales.

10. - Vecteur selon la revendication 9 pour l'utilisation dans le traitement de maladies tumorales en combinaison à une radiothérapie.

11. - Vecteur selon la revendication 9 pour l'utilisation dans le traitement de maladies tumorales en combinaison à une chimiothérapie.
